# EUROPEAN PATENT SPECIFICATION

(11) **EP 4 311 830 B1**
(45) Date of publication and mention of the grant of the patent: **27.08.2025**
(21) Application number: 22187899.4
(22) Date of filing: 29.07.2022
(51) Int. Cl.: C07F 15/00, A61P 35/00

(54) **CISPLATIN ANALOGUE WITH POTENT ANTI-CANCER EFFECTS AND SYNTHESIS THEREOF**
CISPLATINANALOGE MIT POTENTEN ANTI-KREBS-EFFEKTEN UND SYNTHESE DAVON
ANALOGUE DE CISPLATINE AVEC EFFETS ANTI-CANCER PUISSANTS ET SA SYNTHÈSE

(43) Date of publication of application: 31.01.2024
(73) Proprietor: King Faisal Specialist Hospital & Research Centre, 11211 Riyadh (SA)
(72) Inventor: Aboussekhra, Abdelilah, 11211 Riyadh (SA); Al-Jammaz, Ibrahim, 11211 Riyadh (SA); Al-Otaibi, Basem, 11211 Riyadh (SA); Alraouji, Noura N., 11211 Riyadh (SA)
(74) Representative: Engelhard, Markus

(56) References cited:
- US-A1- 2007 286 905
- THANH CHI NGUYEN THI ET AL: "Synthesis and spectral characterization of platinum(II) complexes containing eugenol, a natural allylphenol", JOURNAL OF COORDINATION CHEMISTRY, vol. 70, no. 6, 2 February 2017 (2017-02-02), London, pages 1008 - 1019, XP093015618, ISSN: 0095-8972, DOI: 10.1080/00958972.2017.1281917

## Description

The present invention relates to a compound comprising a cisplatin component and a eugenol component. The present invention further relates to a synthesis method of said compound, pharmaceutical compositions comprising the compound and its medical uses. The present invention further relates to a method of treatment of cancer.

### BACKGROUND OF THE INVENTION

Cisplatin (cis-diamminedichloroplatinum II) is a well-known metal-based DNA damaging chemotherapeutic drug, which has been used for the treatment of different types of cancer. Indeed, cisplatin is effective against various types of tumors, including carcinomas, lymphomas, and sarcomas (Tchounwou *et al.,* 2021). Unfortunately, even low and non-toxic concentrations of cisplatin in the serum could be toxic in the kidneys. Indeed, 30-40% of cisplatin-treated patients develop acute kidney injury (Volarevic *et al.,* 2019). To overcome these clinical limitations, different cisplatin analogues were synthesized and are currently used for the treatment of various types of tumors. These include carboplatin and oxaliplatin, which showed efficiency and less toxicity (Desoize *et al.,* 2002; Ali *et al.,* 2013).

We have recently shown that eugenol, a phenolic natural compound present essentially in clove oil with anti-cancer potential, can potentiate the effect of cisplatin against breast cancer and ovarian cancer cells both *in vitro* and *in vivo* (Islam *et al.,* 2018; Islam *et al.,* 2019). Interestingly, while the simultaneous combination of cisplatin with eugenol was very effective against breast cancer cells, it was ineffective and generated an antagonistic effect against ovarian cancer cells (Islam *et al.,* 2018; Islam *et al.,* 2019). In fact, only the sequential combination (cisplatin followed by eugenol) was effective against ovarian cancer cells (Islam *et al.,* 2019). US 2007/286905 A1 discloses platinum complexes used in for the treatment of cancer.

Thus, there is a need for developing further cisplatin analogues with higher efficiency and less side effects.

There is also a need to have effective drugs against resistant as well as recurrent tumors, which are the most resistant ones. There is also a further need for the treatment of deadly metastatic tumors.

### SUMMARY OF THE INVENTION

According to the present invention this object is solved by a compound comprising a cis-diamminedichloroplatinum II component (cisplatin component) and a 2-methoxy-4-(prop-2-en-1-yl)phenol component (eugenol component) which are covalently connected via a linker.

According to the present invention this object is solved by a method of synthesizing the compound of the present invention comprising
(1) adding eugenol and a base, preferably NaOH, to water;
   and optional, stirring the mixture;
(2) adding cisplatin to the mixture of (1);
   and optional, stirring the mixture;
(3) obtaining the compound, which is preferably a solid, which is more preferably washed and dried.

According to the present invention this object is solved by a pharmaceutical composition comprising
(a) a compound of the present invention or a compound obtained by the method of the present invention;
(b) optional, pharmaceutically acceptable excipient(s) or carrier.

According to the present invention this object is solved by providing the compound of the present invention or the compound obtained by the present invention or the pharmaceutical composition of the present invention for use in medicine.

According to the present invention this object is solved by providing the compound of the present invention or the compound obtained by the present invention or the pharmaceutical composition of the present invention for use in a method of treatment of cancer.

According to the present invention this object is solved by a method of treatment of cancer, comprising
administering to a subject in need thereof a therapeutically amount of a compound of the present invention or a compound obtained by the method of the present invention or the pharmaceutical composition of the present invention.

In yet a further aspect of the present invention, this object is solved by the use of the compound in accordance with the present invention for the manufacture of a medicament for the treatment of cancer.

### DESCRIPTION OF THE PREFERRED EMBODIMENTS OF THE INVENTION

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "18 to 30" should be interpreted to include not only the explicitly recited values of 18 to 30, but also include individual values and sub-ranges within the indicated range. Thus, included in this numerical range are individual values such as 18, 19, 20, 21 .... 29, 30 and sub-ranges such as from 20 to 28, 20 to 25, 21 to 24, 7 to 9 etc. This same principle applies to ranges reciting only one numerical value, such as below 25". Furthermore, such an interpretation should apply regardless of the breadth of the range or the characteristics being described.

### Cisplatin analogues

As outlined above, the present invention provides compounds a cis-diamminedichloroplatinum II component (cisplatin component) and a 2-methoxy-4-(prop-2-en-1-yl)phenol component (eugenol component).

The compound of the present invention comprises a cisplatin component and an eugenol component, which are covalently connected via a linker.

Cisplatin is a chemotherapy medication used to treat a number of cancers. Other names are cisplatinum, platamin, neoplatin, cismaplat, cis-diamminedichloroplatinum II (CDDP). The IUPAC name is: (SP-4-2)-diamminedichloridoplatinum(II). The sum formula is [Pt(NH₃)₂Cl₂] or PtCl₂(NH₃)₂. Cisplatin has the formula:

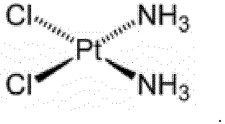

A "cisplatin component" as used herein refers to cisplatin and to cisplatin analogues with the sum formula PtCl(NH₃)₂.

Eugenol is an allyl chain-substituted guaiacol, a member of the allylbenzene class of chemical compounds. It is a colorless to pale yellow, aromatic oily liquid extracted from certain essential oils especially from clove, nutmeg, cinnamon, basil and bay leaf. It is present in concentrations of 80-90% in clove bud oil and at 82-88% in clove leaf oil. The preferred IUPAC name is 2-methoxy-4-(prop-2-en-1-yl)phenol. The sum formula is C₁₀H₁₂O₂. Eugenol has the formula:

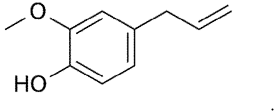

A "eugenol component" as used herein refers to eugenol and to eugenol analogues with the sum formula C₁₀H₁₁O₂.

The compound of the present invention preferably has the general formula I

**Cis - L - Eug** **(I)**

wherein
**Cis** is the cisplatin component,
**L** is a linker, and
**Eug** is the eugenol component.

A "linker" as used herein is preferably a covalent bond between the cisplatin component and the eugenol component.

The compound of the present invention can be obtained by reacting eugenol with cisplatin, preferably in presence of a base.

The compound of the present invention can be called eugenoplatin (EP). The compound of the present invention is an anti-cancer compound.

The compound of the present invention has preferably the sum formula PtCl(NH₃)₂C₁₀H₁₁O₂.

The compound of the present invention also comprises the pharmaceutically active salts.

The compound of the present invention has preferably the formula 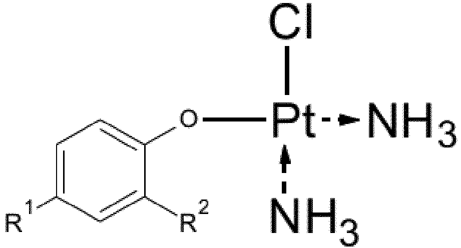 wherein
R¹ is -CH₂-CH=CH₂ or -CH=CH-CH₃, and
R2 is -OCH₃ or -OCH₂-CH₃,
or a pharmaceutically active salt thereof.

In one embodiment, the compound of the present invention has the formula 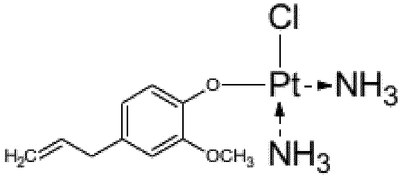 or a pharmaceutically active salt thereof.

### Synthesis method

As outlined above, the present invention provides a method of synthesizing the compound of the present invention.

Said method comprises the following steps
(1) adding eugenol and a base, preferably NaOH, to water;
   and optional, stirring the mixture;
(2) adding cisplatin to the mixture of (1);
   and optional, stirring the mixture;
(3) obtaining the compound, which is preferably a solid, which is more preferably washed and dried.

### - Step (1)

In step (1), eugenol and a base are added to water.

The base is preferably NaOH or KOH.

Preferably, eugenol and the base have a similar molarity / are added in a ratio of about 1:1 by molarity.

The mixture of eugenol and the base is preferably stirred, such as
for about 1 to 4 hours, such as about 2 hours,
at a temperature from about 15 to about 25°C, such as at room temperature.

### - Step (2)

In step (2), cisplatin is added to the mixture of (1).

Preferably, cisplatin and eugenol and the base have a similar molarity / are added in a ratio of about 1:1:1 by molarity.

The mixture of eugenol and the base and cisplatin is preferably stirred, such as
for about 18 to 30 hours, such as about 24 hours,
at a temperature from about 15 to about 25°C, such as at room temperature.

### - Step (3)

In step (3), the compound is obtained.

The compound obtained in step (3) is preferably a solid.

The solid is preferably washed and dried.

For example, a solid is formed and in addition to that a (yellow) solution. The (yellow) solution is removed by centrifugation and the sticky solid is washed three times with methanol, and is isolated by centrifugation, and then dried under vacuum.

Preferably, the obtained compound is characterized by HPLC analysis and/or mass spectroscopy.

### Pharmaceutical composition and medical uses

As outlined above, the present invention provides a pharmaceutical composition comprising
(a) a compound of the present invention or a compound obtained by the method of the present invention;
(b) optional, pharmaceutically acceptable excipient(s) or carrier.

As outlined above, the present invention provides the compound of the present invention or the compound obtained by the method of the present invention or the pharmaceutical composition of the present invention for use in medicine.

The compounds of the present invention show potent anti-cancer activities against various types of cancer cells. Therefore, the compounds have great therapeutic value for the treatment of different types of tumors.

As outlined above, the present invention provides the compound of the present invention or the compound obtained by the method of the present invention or the pharmaceutical composition of the present invention for use in a method of treatment of cancer.

Preferably, the cancer is breast cancer, ovarian cancer, osteosarcoma, colorectal cancer, glioblastoma, leukemia, lymphoma, lung cancer or thyroid cancer.

In one embodiment, the compound of the present invention or the compound obtained by the method of the present invention or the pharmaceutical composition of the present invention is used in combination with at least one further anticancer treatment,
such as chemotherapy and/or immunotherapy.

In one embodiment, where the use is in combination with chemotherapy, the chemotherapeutic agent can be docetaxel, paclitaxel or doxorubicin.

In one embodiment, where the use is in combination with immunotherapy, different immunotherapeutic molecules can be used.

In one embodiment of the compound for use, said method of treatment of cancer comprises
administering to a subject in need thereof a therapeutically effective amount of a compound of the present invention or a compound obtained by the method of the present invention or the pharmaceutical composition of the present invention.

In one embodiment of the compound for use, the step of administering in said method of treatment is via infusion.

In a further aspect, the present invention also relates to the use of the compound in accordance with the present invention for the manufacture of a medicament for the treatment of cancer.

### Treatment method

As outlined above, the present invention provides a method of treatment of cancer.

Said method comprises
administering to a subject in need thereof a therapeutically effective amount of a compound of the present invention or a compound obtained by the method of the present invention or the pharmaceutical composition of the present invention.

A "therapeutic amount" or a "therapeutically effective amount" of a compound of the present invention refers to the amount which has to be administered to a subject in need thereof in order to achieve a desired therapeutic result or outcome. The skilled artisan will be able to determine said therapeutically effective amount and the suitable administration regimen.

Preferably, the cancer is breast cancer, ovarian cancer, osteosarcoma, colorectal cancer, glioblastoma, leukemia, lymphoma, lung cancer or thyroid cancer.

In a preferred embodiment, the administration is via infusion.

In one embodiment, the method of the present invention is carried out in combination with at least one further anticancer treatment,
such as chemotherapy and/or immunotherapy.

### Further description of preferred embodiments

### - Abstract

The invention relates to the synthesis of a cisplatin analogue called eugenoplatin consisting of an association between cisplatin (PtCl₂(NH₃)₂) and eugenol (C₁₀H₁₂O₂). The novel molecule PtCl(NH₃)₂C₁₀H₁₁O₂ shows potent anti-cancer activities against various types of cancer cells.

Therefore, the new compound has great therapeutic value for the treatment of different types of tumors. The steps of the obtention of this anti-cancer cisplatin analogue are as follows:
1. Synthesis of the combined cisplatin and eugenol molecule (eugenoplatin, EP);
2. HLPC and MS characterization of eugenoplatin;
3. Elucidation of the anticancer properties of eugenoplatin;
4. Experiments that show that eugenoplatin targets cancer stem cells; and
5. Experiments that show that eugenoplatin introduces DNA damage in human cells.

### - Results

### 1. Synthesis of the combined cisplatin and eugenol molecule (eugenoplatin, EP)

Eugenol (50.0 mg at 0.304 mM) and NaOH (320 µl of 1 M.0 M NaOH at 0.320 mM) were added to 2.0 mL water, and the mixture was stirred at room temperature for 120 min. Subsequently, cisplatin (92.0 mg at 0.306 mM) was added to the eugenol solution and the mixture was stirred at room temperature for 24 h, the next day a yellow solution was formed in addition to a sticky solid. The yellow solution was removed by centrifugation and the sticky solid was washed three times with 1.0 mL methanol, and was isolated by centrifugation, and then dried under vacuum.

### 2. HLPC and MS characterization of eugenoplatin

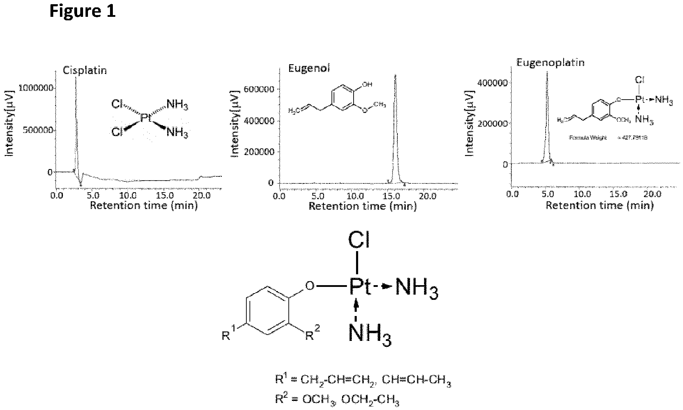

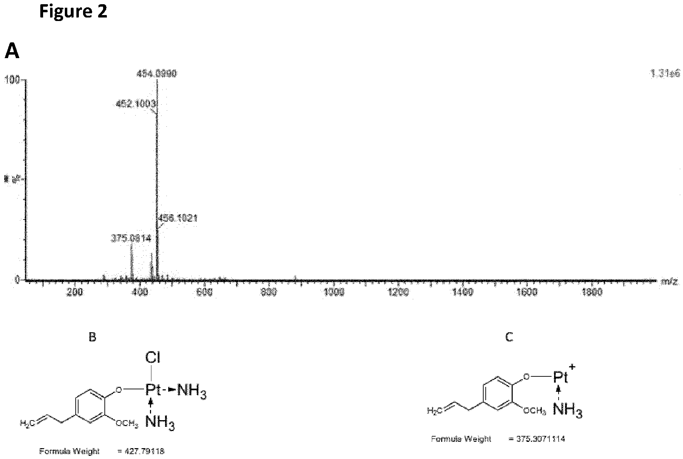

The formed product was first analyzed by HPLC using analytical C18 Columns and dual Detection UV Detector. Figure 1 shows the presence of a 100% pure single peak with no other peaks and no starting material, both at UV=254 nm and at UV=220nm. The novel peak (molecule) had a retention time of 5 min, while the retention time of eugenol and cisplatin are 3 min and 16 min, respectively. This suggests the formation of a third/new molecule with new physical features and an expected molecular weight of 428.0 with the most possible structure as shown in Figure 2B. The Mass Spectroscopy analysis showed one main peak of MS+1=452 (Fig. 2A), which corresponds to the molecule plus sodium (428.0+23=451) (Fig. 2B), and one fragment of MS+1=375, which corresponds to the structure shown in Figure 2C. v

### 3. Anticancer effects of eugenoplatin on different cancer cell lines in vitro

### 3.1 Cytotoxicity

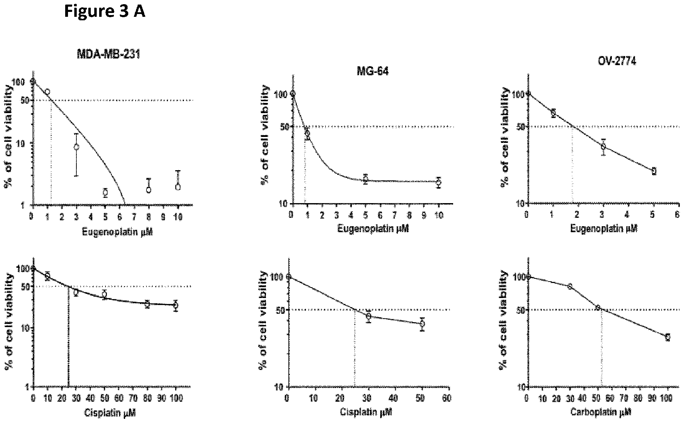

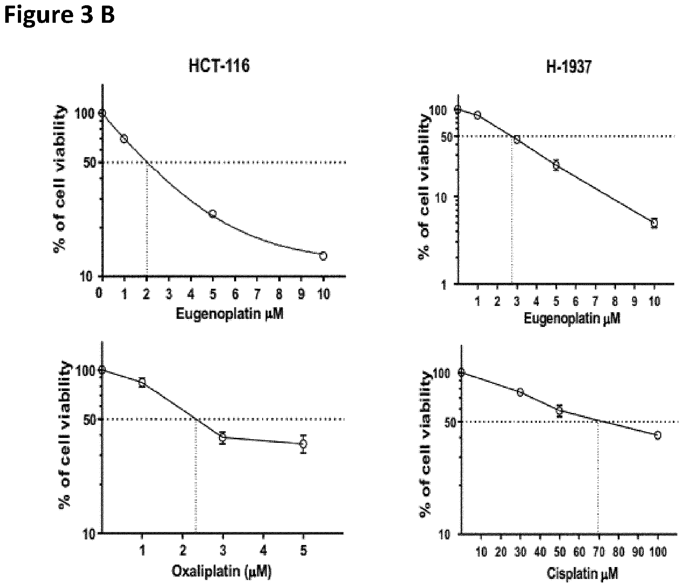

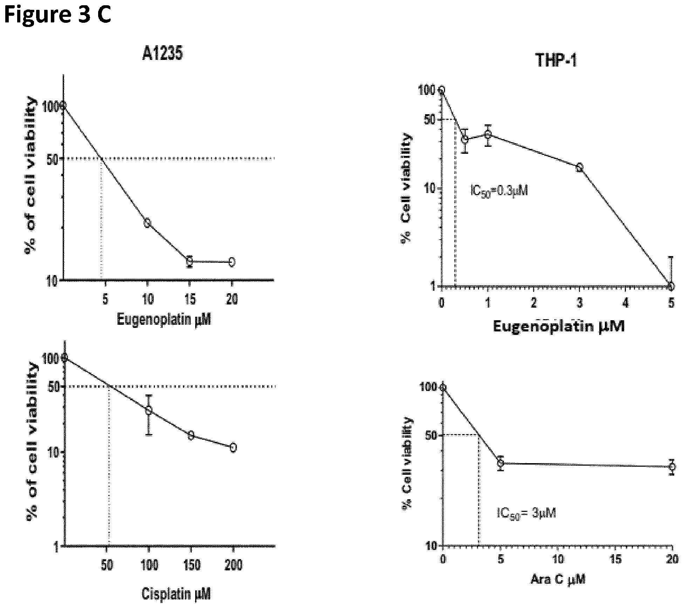

Using the widely used cytotoxicity assay (WST1) we first show that eugenoplatin is highly toxic against cancer cells from different types: breast cancer, ovarian cancer, osteosarcoma, colorectal cancer, glioblastoma, and leukemia with different IC₅₀ (Table 1). Table 1 shows also that the cytotoxic effect of eugenoplatin is higher than that of cisplatin against breast cancer, osteosarcoma and glioblastoma cells. Eugenoplatin showed also higher toxicity against colorectal cancer, ovarian cancer and leukemia cells than oxaloplatin, carboplatin and cytarabine (Ara-C), respectively (Figure 3 and Table 1).

| **Cancer Type** | **Cell lines** | **Eugenoplatin** | **Cisplatin** | **Carboplatin** | **Oxaliplatin** | **Cytarabine Ara-C** |
|---|---|---|---|---|---|---|
| ***Breast*** | MDA-MB-231 | 1.24±0.11 | 22.83±2.13 | - | - | - |
| ***Osteosarcoma*** | MG-64 | 0.88±0.16 | 26±4 | - | - | - |
| ***Lung*** | H-1937 | 2.72±0.19 | 67.75±7.75 | - | - | - |
| ***Glioblastoma*** | A1235 | 4.58±0.08 | 55.75±18.25 | - | - | - |
| ***Thyroid*** | Cal62 | 1.45±0.28 | 25.67±2.19 | - | - | - |
| ***Ovarian*** | OV-2774 | 1.83±0.35 | - | 53.33± 0.67 | - | - |
| ***Colon*** | HCT-116 | 2.2±0.03 | - | - | 3.17± 0.52 | - |
| ***AML*** | THP-1 | 0.3±0.25 | - | - | - | 3±0.05 |

### 3.1 The cytotoxic effect of eugenoplatin is specific to cancer cells

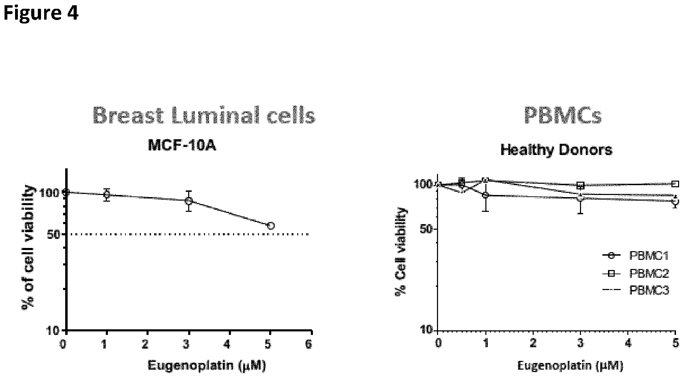

The specificity of anti-cancer drugs is of great importance to limit the side effects of these molecules. Therefore, we tested the cytotoxic effects of eugenoplatin on different types of normal cells. Figure 4 shows that eugenoplatin has only marginal cytotoxicity against breast epithelial cells (MCF-10A), and blood cells (PBMCs from 3 healthy donors). Indeed, eugenoplatin at 5 µM killed only 40% and less than 20% in MCF-10A cells and blood cells, respectively.

### 3.1 Eugenoplatin promotes apoptosis in cancer cells

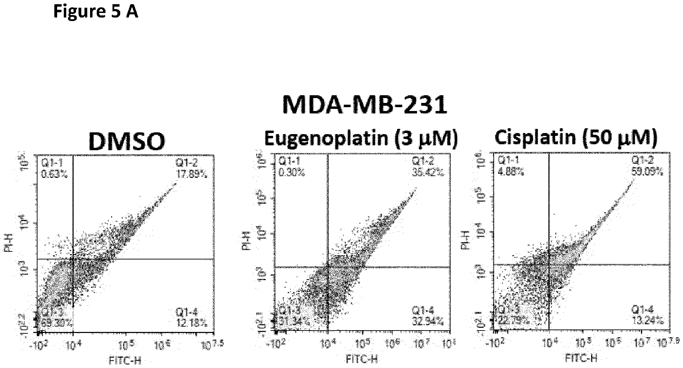

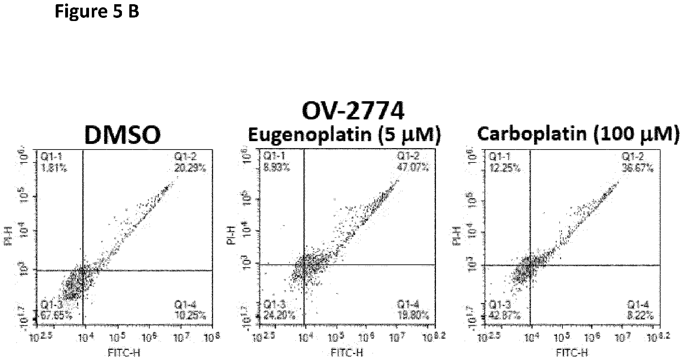

We have used the annexinV/propidium iodide-flow cytometry technique to show that, like cisplatin, eugenoplatin promotes mainly apoptosis in both breast cancer (MDA-MB-231) as well as ovarian cancer (OV-2774) cells (Fig. 5A and 5B). Indeed, at 3 µM and 5 µM of eugenoplatin the proportion of apoptotic cells reached 68% in breast cancer and ovarian cancer cells, respectively (Fig. 5A and 5B). This was confirmed in different cancer cell lines using immunoblotting and antibodies specific for the pro-apoptotic protein PARP, caspase-3, caspase-9.

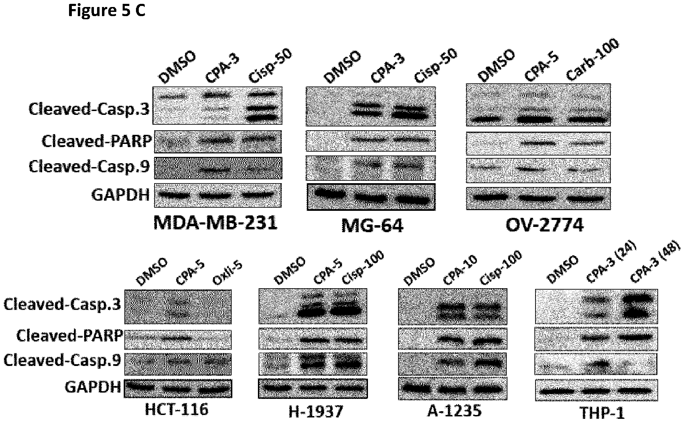

Figure 5C shows that eugenoplatin increased the level of cleaved PARP, caspase-3 and caspase-9 in breast cancer (MDA-MB-231), ovarian cancer (OV-2774), osteosarcoma (MG-64), colon cancer (HCT-116), lung cancer (H-1937), glioblastoma (A1235) and leukemia cells (THP-1). This effect was more efficient than that of cisplatin, carboplatin or oxaliplatin on breast cancer and osteosarcoma cells, ovarian cancer cells, and colorectal cancer cells, respectively (Fig. 5C).

### 4. Eugenoplatin targets cancer stem cells

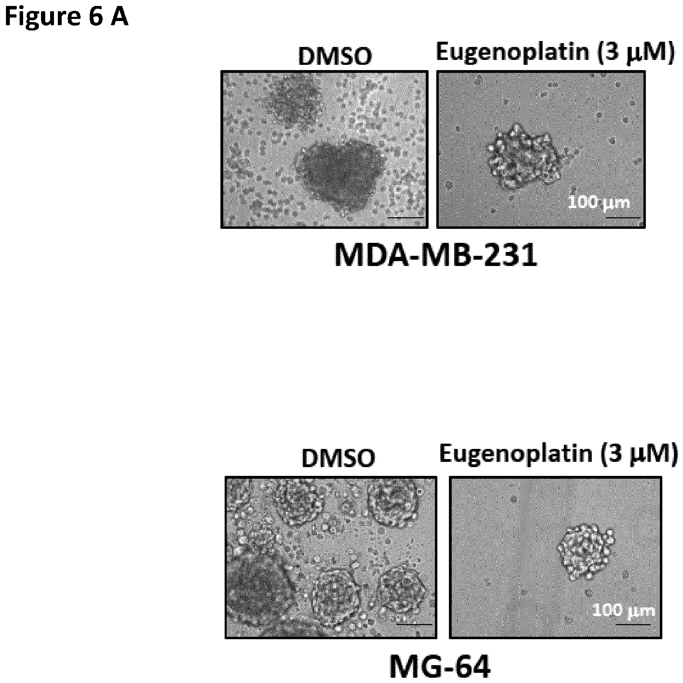

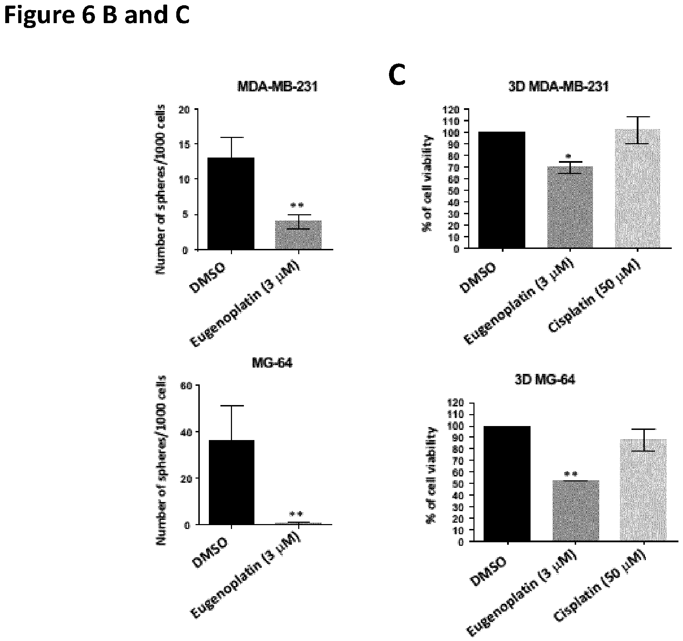

It is well known that cancer stem cells are the most resistant type of cells, which are responsible for recurrence and metastasis (Garcia-Mayea *et al.,* 2020). Therefore, we decided to investigate the effect of eugenoplatin on the self-renewal ability and stemness capacity of cancer cells. To this end, cancer cells were first either sham-treated (DMSO) or challenged with eugenoplatin (3 µM) for 24 h, and then were incubated in 96 well ultra-low attachment plates in the presence of stem cells culture medium. After 10 days, the formed spheroids with a dimeter ≥ 100 µm were counted (Fig. 6A). Figure 6B shows that eugenoplatin treatment reduced by 3-fold and 40-fold the capacity of MDA-MB-231 and MG-64 cells to form tumorespheres, respectively. Furthermore, tumorespheres were first formed, and then they were either sham-treated (DMSO) or challenged with eugenoplatin (3 µM) or cisplatin (50 µM) for 24 h and the cytotoxicity was assessed using WST1. Figure 6C shows that while the proportion of survival CSC was reduced to 70% (MDA-MB-231) and 50% (MG-64) upon treatment with eugenoplatin, it was not affected by cisplatin treatment. This indicates that while cancer stem cells are not sensitive to cisplatin, they showed sensitivity to eugenoplatin.

### 6. Eugenoplatin promotes DNA damage in human fibroblast cells

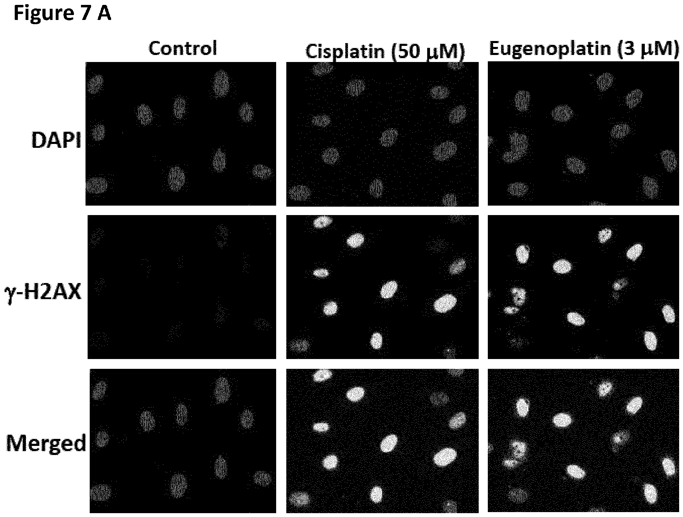

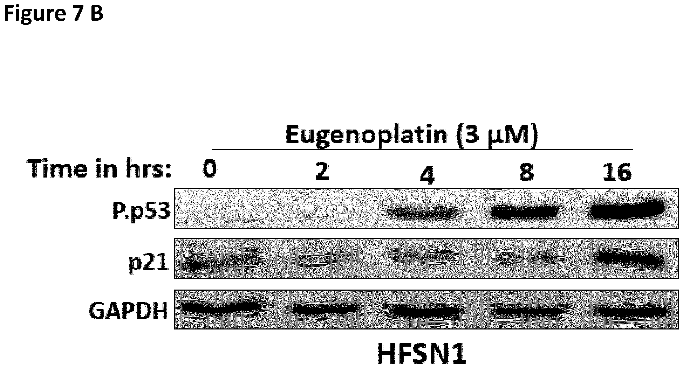

Since cisplatin is a DNA damaging chemotherapeutic drug, we investigated whether eugenoplatin can also promote DNA damage in cells. To this end, human fibroblast cells (HFSN1) were either sham-treated (DMSO) or challenged with cisplatin (50 µM) or eugenoplatin (3 µM) for 24 h, and then the level of the DNA damage sensing protein (y-H2AX) was assessed by immunofluorescence. While no γ-H2AX immunostaining was detected in the control cells, a strong nuclear staining was observed in response to cisplatin, confirming cisplatin-dependent induction of DNA damage (Fig. 7A). Interestingly, cells treated with eugenoplatin also showed γ-H2AX immunostaining at the nucleus (Fig. 7A). This suggests that eugenoplatin can also induce DNA damage in human cells. To confirm this, we assessed the level of the phosphorylated form of p53 (P.p53) in eugenoplatin-treated cells. Figure 7B shows strong increase in the level of P.p53 in a time-dependent manner. This was accompanied by a late increase in the level of the p53 target p21 (Fig. 7B). These results show eugenoplatin-dependent induction of DNA damage in human cells.

The following examples and drawings illustrate the present invention without, however, limiting the same thereto.

### BRIEF DESCRIPTION OF THE DRAWINGS

**Figure 1****.** *HPLC analysis of the three molecules: cisplatin, eugenol and eugenoplatin* using analytical C18 Columns and dual Detection UV Detector.
**Figure 2****.** *Mass Spectroscopy analysis of eugenoplatin.*
   (A) Mass Spectroscopy analysis of the newly synthesized eugenoplatin molecule.
   (B) The molecular formula and the corresponding weight of eugenoplatin.
   (C) The molecular formula and the weight of the molecule corresponding to the peak 375.0814.
**Figure 3****.** *Eugenoplatin is highly cytotoxic against various types of cancer.* The indicated cells, namely MDA-MB-231, MG-64, and OV-2774 in Figure 3A, HCT-116, and H-1937 in Figure 3B, A1235 and THP-1 in Figure 3C, were treated with the indicated concentrations of eugenoplatin or cisplatin, carboplatin, oxaliplatin, AraC as indicated, and then the cytotoxic effects of these drugs was assessed by the WST1 assay. Error bars represent mean±S.D (n=3). The IC₅₀ are indicated by lines in dots.
**Figure 4****.** *Eugenoplatin is not cytotoxic against normal human cells.*
   Breast luminal cells (MCF-10A) and blood cells (PBMCs of healthy donors') were treated with the indicated concentrations of eugenoplatin, and then the cytotoxic effect was assessed by the WST1 assay. Error bars represent mean±S.D (n=3).
**Figure 5****.** *Eugenoplatin promotes apoptosis in tumor cells from various types of cancer.*
   (A) and (B) MDA-MB-231 and OV-2774 cells, respectively, were treated with eugenoplatin or cisplatin/carboplatin as indicated. Cell death was then assessed by annexinV/propidium iodide-associated with flow cytometry.
   (C) The indicated cells, namely MDA-MB-231, MG-64, OV-2774, HCT-116, H-1937, A1235 and THP-1, were treated as indicated, and then whole cell lysates were prepared and used for immunoblotting analysis using antibodies against the indicated proteins.
**Figure 6****.** *Eugenol targets cancer stem cells.*
   Cells (2000) were first either sham-treated (DMSO) or challenged with eugenoplatin (3 µM) for 24 h, and then were incubated in 96 well ultra-low attachment plates in the presence of stem cells culture medium. After 10 days, the formed spheroids with a dimeter ≥ 100 µM were counted.
   (A) Images of the formed spheroids.
   (B) Histogram showing the number of formed spheroids. Error bars represent mean±S.D (n=3).
   (C) Cells were first incubated in 96 well ultra-low attachment plates in the presence of stem cells culture medium. After 10 days, the formed spheroids with a dimeter ≥ 100 µM were either sham-treated (DMSO) or challenged with eugenoplatin (3 µM) or cisplatin (50 µM) for 24 h and the cytotoxicity was assessed using WST1. Error bars represent mean±S.D (n=3).
**Figure 7****.** Eugenoplatin triggers DNA damage in human cells.
   (A) Human fibroblast cells (HFSN1) were either sham-treated (DMSO) or challenged with cisplatin (50 µM) or eugenoplatin (3 µM) for 24 h, and then the level of the DNA damage sensing protein (y-H2AX) was assessed by immunofluorescence.
   (B) Cells were either sham-treated or challenged with cisplatin or eugenoplatin (3 µM) for the indicated periods of time, and then cell lysates were prepared and used for immunoblotting analysis using antibodies against the indicated proteins.

### EXAMPLES

### 1. Materials and Methods

### 1.1 Cytotoxicity assay (WST1)

5,000 cells/well were seeded in 96-well plates with appropriate culture media. After cells treatment, WST-1 reagent (Sigma-Aldrich) was added to each well according to the manufacturer's instructions. These experiments were performed in triplicates and were repeated several times.

### 1.2 Apoptosis analysis by Annexin V/Flow Cytometry

Cells were harvested, centrifuged and stained with propidium iodide (PI) or PI and Alexa Flour 488 annexin V (Molecular Probes, Eugene), and then were analysed by flow cytometry.

### 1.3 Immunofluorescence

HFSN1 cells were fixed in formaldehyde (4%) for 19 minutes and blocked with Goat serum (5%), triton X (0.3%) and 1% sodium azide (5%) for 1 hour. The slides were then stained overnight at 4 °C with γ-H2AX antibody (pSer139) (Novusibio) that diluted in BSA (1%), triton X (0.3%) and 1 % sodium azide (5%), and subsequently incubated with alexa flour 594-conjugated goat anti-rabbit IgG and DAPI for 1 hour. Images were acquired using fluorescence microscope (Zeiss).

### 1.4 3D spheroid assay

Cells were seeded in 96 well ultra-low attachment plate at a density of 1000 viable cells/well. Cells were cultured in 171 medium supplemented with 1% ABM, 2% B-27, 20 ng/mL EGF, 500 ng/ml HC, 4% FBS and 5 µg/ml insulin. Cells were incubated for 10 days at 37°C under 5% CO_{2.} Mammospheres with a diameter of ≥100 µm were counted using OPTIKA light microscope.

The features disclosed in the foregoing description, in the claims and/or in the accompanying drawings may, both separately and in any combination thereof, be material for realizing the invention in diverse forms thereof.

### REFERENCES

Ali I, Wani WA, Saleem K, Haque A. Platinum compounds: a hope for future cancer chemotherapy. Anticancer Agents Med Chem. 2013; 13(2):296-306.

Desoize B, Madoulet C. Particular aspects of platinum compounds used at present in cancer treatment. Crit Rev Oncol Hematol. 2002; 42(3):317-25.

Garcia-Mayea Y, Mir C, Masson F, Paciucci R, ME LL. Insights into new mechanisms and models of cancer stem cell multidrug resistance. Semin Cancer Biol. 2020; 60:166-80.

Islam SS, Al-Sharif I, Sultan A, Al-Mazrou A, Remmal A, Aboussekhra A. Eugenol potentiates cisplatin anti-cancer activity through inhibition of ALDH-positive breast cancer stem cells and the NF-kappaB signaling pathway. Mol Carcinog. 2018; 57(3):333-46.

Islam SS, Aboussekhra A. Sequential combination of cisplatin with eugenol targets ovarian cancer stem cells through the Notch-Hes1 signalling pathway. J Exp Clin Cancer Res. 2019; 38(1):382.

Tchounwou PB, Dasari S, Noubissi FK, Ray P, Kumar S. Advances in Our Understanding of the Molecular Mechanisms of Action of Cisplatin in Cancer Therapy. J Exp Pharmacol. 2021; 13:303-28.

Volarevic V, Djokovic B, Jankovic MG, Harrell CR, Fellabaum C, Djonov V, Arsenijevic N. Molecular mechanisms of cisplatin-induced nephrotoxicity: a balance on the knife edge between renoprotection and tumor toxicity. J Biomed Sci. 2019; 26(1):25.

## Claims

1. A compound comprising a cis-diamminedichloroplatinum II component (cisplatin component) and a 2-methoxy-4-(prop-2-en-1-yl)phenol component (eugenol component) which are covalently connected via a linker.

2. The compound of claim 1 having the general formula I
**Cis - L - Eug** **(I)**
wherein
**Cis** is the cisplatin component,
**L** is a linker, and
**Eug** is the eugenol component.

3. The compound of claim 1 or 2 having the sum formula PtCl(NH₃)₂C₁₀H₁₁O₂.

4. The compound of any one of claims 1 to 3 having the formula 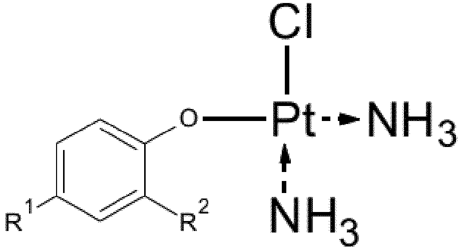 wherein
R¹ is -CH₂-CH=CH₂ or -CH=CH-CH₃, and
R2 is -OCH₃ or -OCH₂-CH₃,
or a pharmaceutically active salt thereof.

5. Method of synthesizing the compound of any one of claims 1 to 4 comprising
(1) adding eugenol and a base, preferably NaOH, to water;
and optional, stirring the mixture;
(2) adding cisplatin to the mixture of (1);
and optional, stirring the mixture;
(3) obtaining the compound, which is preferably a solid, which is more preferably washed and dried.

6. A pharmaceutical composition comprising
(a) a compound of any one of claims 1 to 4 or a compound obtained by the method of claim 5;
(b) optional, pharmaceutically acceptable excipient(s) or carrier.

7. The compound of any one of claims 1 to 4 or the compound obtained by the method of claim 5 or the pharmaceutical composition of claim 6 for use in medicine.

8. The compound of any one of claims 1 to 4 or the compound obtained by the method of claim 5 or the pharmaceutical composition of claim 6 for use in a method of treatment of cancer.

9. The compound for use according to claim 8, wherein the cancer is breast cancer, ovarian cancer, osteosarcoma, colorectal cancer, glioblastoma, leukemia, lymphoma, lung cancer or thyroid cancer.

10. The compound for use according to claim 8 or 9, in combination with at least one further anticancer treatment,
such as chemotherapy and/or immunotherapy.

11. The compound for use according to any of claims 8 - 10, wherein said method of treatment of cancer comprises
administering to a subject in need thereof a therapeutically effective amount of said compound of any one of claims 1 to 4 or a compound obtained by the method of claim 5 or the pharmaceutical composition of claim 6.

12. The compound for use according to claim 11, wherein administering in said method of treatment is via infusion.

## Patentansprüche

1. Verbindung, umfassend eine cis-Diammindichlorplatin-II-Komponente (Cisplatin-Komponente) und eine 2-Methoxy-4-(prop-2-en-1-yl)phenol-Komponente (Eugenol-Komponente), die über einen Linker kovalent verbunden sind.

2. Verbindung nach Anspruch 1, die die allgemeine Formel I hat
**Cis - L - Eug** **(I),**
wobei
**Cis** die Cisplatin-Komponente ist,
**L** ein Linker ist, und
**Eug** die Eugenol-Komponente ist.

3. Verbindung nach Anspruch 1 oder 2, die die Summenformel PtCl(NH₃)₂C₁₀H₁₁O₂ hat.

4. Verbindung nach einem der Ansprüche 1 bis 3, die die Formel 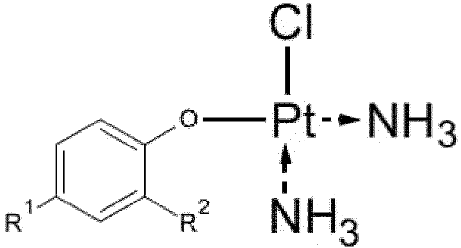 hat, wobei
R¹ -CH₂-CH=CH₂ oder -CH=CH-CH₃ ist, und
R2 -OCH₃ oder -OCH₂-CH₃ ist,
oder ein pharmazeutisch wirksames Salz davon.

5. Verfahren zur Synthese der Verbindung nach einem der Ansprüche 1 bis 4, umfassend
(1) Hinzufügen von Eugenol und einer Base, vorzugsweise NaOH, zu Wasser;
und optional, Rühren der Mischung;
(2) Hinzufügen von Cisplatin zu der Mischung aus (1);
und optional, Rühren der Mischung;
(3) Erhalten der Verbindung, die vorzugsweise ein Feststoff ist, der noch bevorzugter gewaschen und getrocknet wird.

6. Pharmazeutische Zusammensetzung, umfassend
(a) eine Verbindung nach einem der Ansprüche 1 bis 4 oder eine Verbindung, die durch das Verfahren nach Anspruch 5 erhalten wird;
(b) gegebenenfalls, pharmazeutisch verträgliche(n) Hilfsstoff(e) oder Träger.

7. Verbindung nach einem der Ansprüche 1 bis 4 oder Verbindung, die durch das Verfahren nach Anspruch 5 erhalten wird, oder pharmazeutische Zusammensetzung nach Anspruch 6 zur Verwendung in der Medizin.

8. Verbindung nach einem der Ansprüche 1 bis 4 oder Verbindung, die durch das Verfahren nach Anspruch 5 erhalten wird, oder pharmazeutische Zusammensetzung nach Anspruch 6 zur Verwendung in einem Verfahren zur Behandlung von Krebs.

9. Verbindung zur Verwendung nach Anspruch 8, wobei der Krebs Brustkrebs, Eierstockkrebs, Osteosarkom, Darmkrebs, Glioblastom, Leukämie, Lymphom, Lungenkrebs oder Schilddrüsenkrebs ist.

10. Verbindung zur Verwendung nach Anspruch 8 oder 9 in Kombination mit mindestens einer weiteren Krebsbehandlung,
wie beispielsweise Chemotherapie und/oder Immuntherapie.

11. Verbindung zur Verwendung nach einem der Ansprüche 8-10, wobei das Verfahren zur Behandlung von Krebs das Verabreichen einer therapeutisch wirksamen Menge der Verbindung nach einem der Ansprüche 1 bis 4 oder einer Verbindung, die durch das Verfahren nach Anspruch 5 erhalten wird, oder der pharmazeutischen Zusammensetzung nach Anspruch 6 an einen Patienten, der diese benötigt, umfasst.

12. Die Verbindung zur Verwendung nach Anspruch 11, wobei das Verabreichen in dem Verfahren zur Behandlung durch Infusion erfolgt.

## Revendications

1. Composé comprenant un composant cis-diamminedichloroplatine II (composant cisplatine) et un composant 2-méthoxy-4-(prop-2-én-1-yl)phénol (composant eugénol) qui sont liés de manière covalente par un lieur.

2. Composé selon la revendication 1 ayant la formule générale I :
**Cis - L - Eug** **(1)**
dans lequel
Cis représente le composant cisplatine,
L représente un lieur, et
Eug représente le composant eugénol.

3. Composé selon la revendication 1 ou 2 ayant la formule PtCl(NH₃)₂C₁₀H₁₁O₂.

4. Composé selon l'une quelconque des revendications 1 à 3 ayant la formule 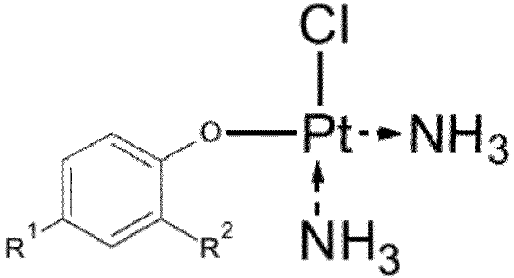 dans lequel
R¹ représente -CH₂-CH=CH₂ ou -CH=CH-CH₃, et
R2 représente -OCH₃ ou -OCH₂-CH₃,
ou un sel pharmaceutiquement actif de celui-ci.

5. Procédé de synthèse du composé selon l'une quelconque des revendications 1 à 4, comprenant :
(1) l'ajout d'eugénol et d'une base, de préférence NaOH, à de l'eau ;
et éventuellement l'agitation du mélange ;
(2) l'ajout de cisplatine au mélange de (1) ;
et éventuellement l'agitation du mélange ;
(3) l'obtention du composé, qui est de préférence un solide, qui est plus préférablement lavé et séché.

6. Composition pharmaceutique comprenant
(a) un composé selon l'une quelconque des revendications 1 à 4 ou un composé obtenu par le procédé selon la revendication 5 ;
(b) un ou plusieurs excipients ou véhicules pharmaceutiquement acceptables, facultatifs.

7. Composé selon l'une quelconque des revendications 1 à 4, ou composé obtenu par le procédé selon la revendication 5 ou composition pharmaceutique selon la revendication 6 pour une utilisation en médecine.

8. Composé selon l'une quelconque des revendications 1 à 4, ou composé obtenu par le procédé selon la revendication 5 ou composition pharmaceutique selon la revendication 6 pour une utilisation dans une méthode de traitement d'un cancer.

9. Composé pour une utilisation selon la revendication 8, dans lequel le cancer est un cancer du sein, un cancer de l'ovaire, un ostéosarcome, un cancer colorectal, un glioblastome, une leucémie, un lymphome, un cancer du poumon ou un cancer de la thyroïde.

10. Composé pour une utilisation selon la revendication 8 ou 9, en association avec au moins un autre traitement anticancéreux,
tel qu'une chimiothérapie et/ou une immunothérapie.

11. Composé pour une utilisation selon l'une quelconque des revendications 8 à 10, dans lequel ladite méthode de traitement du cancer comprend
l'administration à un sujet en ayant besoin d'une quantité thérapeutiquement efficace dudit composé selon l'une quelconque des revendications 1 à 4 ou d'un composé obtenu par le procédé selon la revendication 5 ou de la composition pharmaceutique selon la revendication 6.

12. Composé selon la revendication 11, dans lequel l'administration dans ladite méthode de traitement se fait par perfusion.
